# EUROPEAN PATENT SPECIFICATION

(11) **EP 3 624 736 B1**
(45) Date of publication and mention of the grant of the patent: **08.07.2026**
(21) Application number: 18772936.3
(22) Date of filing: 18.05.2018
(51) Int. Cl.: A61F 2/32, A61F 2/38, A61F 2/40, A61F 2/42, A61F 2/30, A61B 5/11, A61B 5/00, A61B 34/10, A61B 34/20

(54) **SYSTEMS AND METHODS FOR DETERMINING THE POSITION AND ORIENTATION OF AN IMPLANT FOR JOINT REPLACEMENT SURGERY**
SYSTEME UND VERFAHREN ZUR BESTIMMUNG DER POSITION UND ORIENTIERUNG EINES IMPLANTATS FÜR GELENKERSATZOPERATIONEN
SYSTÈMES ET PROCÉDÉS POUR DÉTERMINER LA POSITION ET L'ORIENTATION D'UN IMPLANT POUR UNE CHIRURGIE DE REMPLACEMENT D'ARTICULATION

(30) Priority: 18.05.2017 US 201762508252 P
(43) Date of publication of application: 25.03.2020
(60) Divisional application: 26180543.6
(73) Proprietor: Smith & Nephew, Inc., Memphis, TN 38116 (US)
(72) Inventor: AUSTIN, Gene Edward, Bartlett, Tennessee 38135 (US)
(74) Representative: Appleyard Lees IP LLP
(86) International application number: PCT/US2018/033458
(87) International publication number: WO 2018/213749

(56) References cited:
- CA-A1- 2 954 679
- US-A1- 2004 106 861
- US-A1- 2007 066 917
- US-A1- 2012 029 345
- US-A1- 2013 211 259
- US-A1- 2015 045 700
- US-A1- 2016 278 868

## Description

### CLAIM OF PRIORITY

This application claims the benefit of priority to U.S. Provisional Application No. 62/508,252 titled "Systems and Methods for Determining the Position and Orientation of an Implant for Joint Replacement Surgery," filed May 18, 2017.

### TECHNICAL FIELD

The present disclosure is generally related to apparatuses, systems and methods for computer-aided orthopedic surgery. More specifically, the present disclosure is related to automatically determining the position and orientation of an implant for a patient in advance of joint replacement surgery.

### BACKGROUND

The use of computers, robotics, and imaging to aid orthopedic surgery is known in the art. There has been a great deal of study and development of computer-aided navigation and robotic systems used to guide surgical procedures. For example, a precision freehand sculptor employs a robotic surgery system to assist the surgeon in accurately cutting a bone into a desired shape. In procedures such as total hip replacement (THR), computer-aided surgery techniques have been used to improve the accuracy and reliability of the surgery. Orthopedic surgery guided by images has also been found useful in preplanning and guiding the correct anatomical position of displaced bone fragments in fractures, along a good fixation by osteosynthesis.

Cut guides or cutting blocks can be used in an orthopedic surgical procedure to assist a surgeon in cutting or modifying some portions of a target bone. For example, in joint replacement surgeries, such as THR or total knee replacement (TKR), the preparation of the bones can involve temporarily affixing saw guide cutting blocks to the bones so that a reciprocating saw blade can be held steady along its intended path. Placement of these blocks can be guided by manual instrumentation or through the use of jigs.

The positioning of cutting blocks can be a time consuming and complicated process, which is critical to positive outcomes for the patient. Mechanisms that allow the cutting blocks to be adjusted within the required workspace are complex, and require high machining tolerances, adding to the costs and complexity of these instrument systems.

Manual alignment, in particular, can be cumbersome and is limited to information obtainable through mechanical and visual referencing means. The instruments used to manually align cutting blocks cannot fully capture the 3D shape of the bones, nor can they adequately capture information about kinematics of the joint or soft tissue tension or laxity. Such instruments are also expensive to create and manage, and result in significant operational costs to maintain and clean such instruments. These costs increase the burden of managing cutting blocks. Mechanical referencing instruments can add to the burden of managing cut guides or cutting blocks.

Document CA2954679A1 discloses a system and methods for bone and tissue reconstruction. In order to carry out this reconstruction, the system and associated methods utilizes anatomical images representative of one or more persons. These images are processed to create a virtual three dimensional (3D) tissue model or a series of virtual 3D tissue models mimicking the proper anatomy in question. Thereafter, the system and associated methods are utilized to create a mold and/or other devices (e.g., fixation devices, grafting devices, patient-specific implants, patient-specific surgical guides) for use with reconstructive surgery.

### SUMMARY

The invention is defined in independent claims 1 and 6.

### BRIEF DESCRIPTION OF THE DRAWINGS

Aspects, features, benefits and advantages of the embodiments described herein will be apparent with regard to the following description, appended claims, and accompanying drawings where:
FIG. 1 depicts an illustrative implant device that can be attached to a bone as part of a surgical procedure.
FIGS. 2A and 2B depict illustrative devices for tracking movement within a joint in accordance with certain embodiments.
FIG. 3A depicts an illustrative detection and/or navigation system in accordance with certain embodiments.
FIG. 3B depicts an illustrative block diagram of the detection and/or navigation system as shown in FIG. 3A.
FIG. 4 depicts an illustrative flow diagram of an illustrative method of assessing and determining information pertaining to a patient in accordance with an embodiment.
FIG. 5 depicts an illustrative flow diagram for placing and orienting an implant in accordance with an embodiment.
FIG. 6 depicts an alternative illustrative flow diagram for placing and orienting an implant in accordance with an embodiment.

### DETAILED DESCRIPTION

As used in this document, the singular forms "a," "an," and "the" include plural references unless the context clearly dictates otherwise. Unless defined otherwise, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention. As used in this document, the term "comprising" means "including, but not limited to."

The embodiments of the present teachings described below are not intended to be exhaustive or to limit the teachings to the precise forms disclosed in the following detailed description. Rather, the embodiments are chosen and described so that others skilled in the art may appreciate and understand the principles and practices of the present teachings.

This disclosure describes certain embodiments of navigated surgical systems and processes for determining orientation of a tool or an implant component based upon collected clinical measures and biomechanical information and optimized simulation based upon the collected data.

For the purposes of this specification, the term "implant" is used to refer to a prosthetic device or structure manufactured to replace or enhance a biological structure, either permanently or on a trial basis. For example, in a knee replacement procedure, an implant can be placed on one or both of the tibia and femur. While the term "implant" is generally considered to denote a man-made structure (as contrasted with a transplant), for the purposes of this specification, an implant can include a biological tissue or material transplanted to replace or enhance a biological structure.

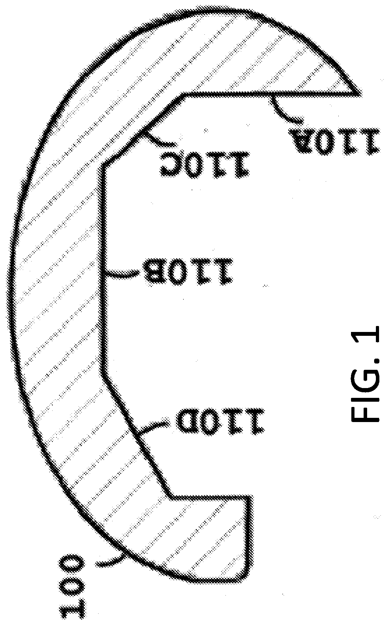

FIG. 1 depicts an illustrative implant component or device 100 that can be used in knee replacement surgery. In certain embodiments, the implant device 100 is placed at the distal end of the patient's femur. The implant device 100 may be attached to a surface formed from a bone cut on a post-operative bone (not shown). The implant device 100 may include an interfacing surface sized and shaped to be in close contact with the post-operative surface of the target bone. The interfacing surface can include multiple facets 110A-D oriented in conformity with the cutting planes, respectively.

When a surgeon places the implant device 100 in a joint, the surgeon must often create surfaces, holes or channels in the bone that mirror protrusions on the implant device and keep it affixed to the bone in the proper position. For optimal fit, the surgeon can use a cutting tool, such as a reciprocating saw or a spinning bur, to shape the bones to receive corresponding implant devices with as little gap as possible. In some cases, a sagittal reciprocating saw may be used with a cutting block in order to cut the bones to the desired shape.

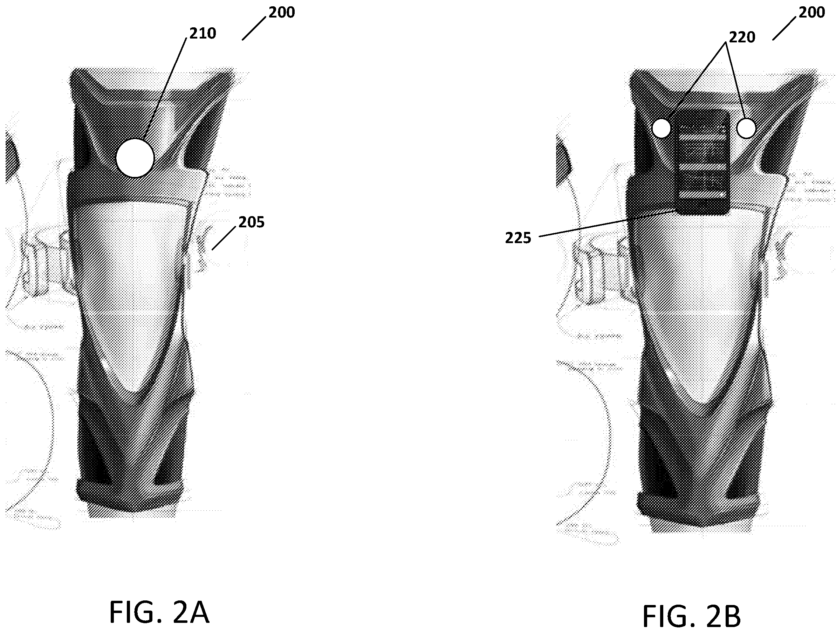

FIGS. 2A and 2B depict illustrative devices for tracking movement within a joint in accordance with certain embodiments. As shown in FIG. 2A, the device 200 may include a sleeve 205 and one or more position trackers 210. As shown in FIG. 2B, the sleeve 205 may include one or more sensors 220 and a communication system 225.

The sleeve 205 may be configured to be securely positioned with respect to a portion of a patient's body. As shown, the sleeve 205 may be configured to be securely positioned in proximity to a patient's knee 202. In alternate embodiments, a sleeve designed according to the teachings described herein may be configured to be securely positioned in proximity to a different joint of a patient, such as a patient's hip, ankle, wrist, elbow or the like. In yet another embodiment, a similar sleeve designed according to the teachings described herein may be configured to be securely positioned in proximity to a portion of a patient's extremity, such as a forearm, upper arm, upper leg, lower leg, or the like.

In an embodiment, the sleeve 205 may include an opening 207 configured to receive a portion of the joint, thereby allowing the joint to move while the sleeve is worn by the patient. In addition, the opening 207 may enable a user to determine a proper position for the sleeve 205. In an alternate embodiment, the sleeve 205 may be substantially tubular (i.e., without an opening). A tubular sleeve 205 may be used, in particular, for a sleeve that is to be positioned in proximity to a portion of a patient's extremity.

In an embodiment depicted in FIG. 2A, the sleeve 205 may include one or more position trackers 210. The one or more position trackers 210 may be used to determine the position and/or orientation of the sleeve 205 and/or of one or more of the patient's bones, ligaments, muscles, soft tissue, or the like in three-dimensional space as the patient's extremity moves. For example, the sleeve 205 may be worn while the patient ambulates on a treadmill. In this manner, the position of one or more bones, ligaments, muscles, or soft tissue in and around the knee may be tracked during ambulation to determine movement during the patient's normal gait. This information may be used to determine, for example and without limitation, patient kinematics, abnormal gait mechanics, stress points within the knee based on the gait, or the like in advance of performing surgery upon the patient's knee. In an embodiment, information pertaining to the patient's gait may be provided to a manufacturer of an implant device to assist in determining an appropriate implant device for the patient or to determine an optimal implant location. For example, information retrieved from the one or more position trackers 210 may be sent to a processing center operated by or on behalf of Smith & Nephew, Inc. of Memphis, TN to assist in the selection or manufacture of an artificial knee implant for a patient.

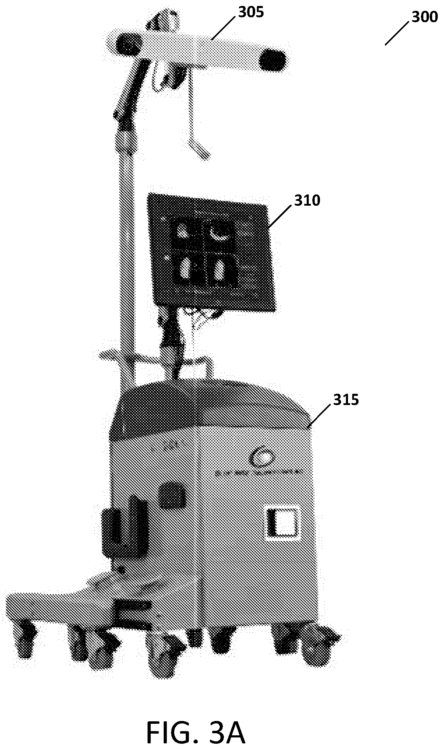

In an embodiment, the one or more position trackers 210 may be tracked by a camera (such as infrared camera 310 further discussed in reference to FIG. 3A) that optically detects the position trackers 210 and communicates that information to a computer system. The location and orientation of the position trackers 210 may be known based on a registration process and based on known characteristics of the sleeve 205.

In an embodiment depicted in FIG. 2B, the sleeve 205 may include one or more sensors 220 and a communication system 225. The one or more sensors 220 may be used to determine the position and/or orientation of the sleeve 205 and/or of one or more of the patient's bones, ligaments, muscles, soft tissue, or the like in three-dimensional space as the patient's extremity moves. In some examples, the one or more sensors 220 can include triple-axis accelerometers configured to measure relative position information and movement data related to the sleeve 205 as the sleeve moves while being worn by the patient. In certain implementations, the one or more sensors can include one or more inertial measurement units (IMUs) that include, for example, one or more accelerometers, gyroscopes, and magnetometers. As with the embodiment described above in reference to FIG. 2A, the sleeve 205 may be worn while the patient ambulates on a treadmill to track the position of one or more bones, ligaments, muscles, or soft tissue in and around the knee.

The communication system 225 may receive the position and/or movement information and transmit the position information to a remote location via the communication interface 225. In an embodiment, the communication interface 225 may include a wireless transmitter operating under any wireless protocol. For example and without limitation, the wireless transmitter may operate using a Bluetooth, cellular, 802.11, NFC, or other communication protocol. In an embodiment, the information may be transmitted to a processing center operated by or on behalf of Smith & Nephew, Inc. of Memphis, TN to assist in the selection or manufacture of an artificial knee implant for the patient. In an alternate embodiment, the information may be transmitted to a local computer system that retransmits the information to a remote location.

FIG. 3A depicts an illustrative detection and/or navigation system in accordance with certain embodiments. As shown in FIG. 3A, the detection and/or navigation system 300 may include a tracking device 305. In some embodiments, the detection and/or navigation system 300 may further include a display 310 and a processing system 315.

In an embodiment, the tracking device 305 may include an infrared camera that identifies the location of position trackers, such as 210 in FIG. 2A, to determine the position and orientation of the patient's extremity during, for example, an ambulation process and/or a surgical procedure. The position trackers 210 and the tracking device 305 can be used to identify and provide data relevant to the precise location of the bones in the knee joint. In certain embodiments, the tracking device 305 can detect tracking spheres located on the position trackers 210 in order to gather location data regarding a patient's femur, tibia, or other bone.

In an embodiment, a detection and/or navigation system 300 may also be used in conjunction with a surgical device having a plurality of position trackers (not shown) to enable the accurate placement and orientation of an implant device in accordance with the teachings herein. For example, movement and/or tracking data collected during ambulation may be processed to identify specifications for an implant device. The detection and/or navigation system 300 may be used during the surgical procedure to guide and direct the surgeon placing the implant within the patient's joint.

In such an embodiment, the display 310 of the detection and/or navigation system 300 may be used to provide feedback information regarding various aspects of the surgical procedure, such as the orientation of the surgical device, the proper location of the implant device, or the like. Further, the processing device 315 of the detection and/or navigation system 300 may be used to determine the position and orientation of the surgical device with respect to a patient in substantially real time. Additional features and description of the detection and/or navigation system 300 may be found in, for example, U.S. Patent No. 6,757,582 and U.S. Patent No. 8,961,536, both of which are incorporated herein by reference in their entireties.

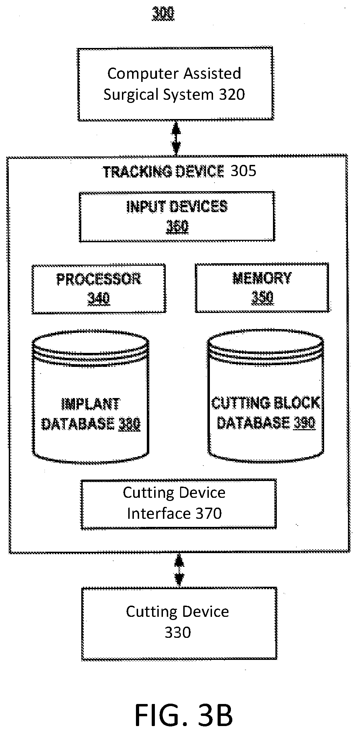

FIG. 3B is a block diagram of the detection and/or navigation system 300 as shown in FIG. 3A. The detection and/or navigation system 300 can be configured to aid a surgeon with various information during a surgical procedure that is consistent with a predetermined surgical plan. The detection and/or navigation system 300 can utilize additional information than traditional systems including, for example, intraoperatively and/or preoperatively defined data related to range of motion capture, and intraoperatively and/or preoperatively defined anatomic landmarks.

The surgical navigation system 300 can configure and implement a tracking device 305 that is connected to a computer assisted surgical (CAS) system 320 that is configured to generate the surgical plan and monitor various activity during the surgical procedure. The tracking device 305 can align and/or control a cutting device 325 for resecting one or more bones in accordance with the surgical plan as generated by the CAS system 320.

The tracking device 305 and/or the CAS system 320 can include one or more processors and memory devices, as well as various input devices, output devices, communication interfaces, and/or other types of devices. The tracking device 305 and/or the CAS system 320 can also include a combination of hardware and software.

The tracking device 305 and/or the CAS system 320 can implement and utilize one or more program modules or similar sets of instructions contained in a computer readable medium or memory (e.g., memory 350) for causing a processing device (e.g., processor 340) to perform one or more operations. Generally, program modules include routines, programs, objects, components, data structures, etc., that perform particular tasks or implement particular abstract data types.

The tracking device 305 and/or the CAS system 320 can be implemented by one or more computing devices such as computers, PCs, server computers configured to provide various types of services and/or data stores in accordance with aspects of the described subject matter. Exemplary server computers can include, without limitation: web servers, front end servers, application servers, database servers, domain controllers, domain name servers, directory servers, and/or other suitable computers. Components of tracking device 305 and/or the CAS system 320 can be implemented by software, hardware, firmware or a combination thereof.

The tracking device 305 can include the processor 340, memory 350, input devices 360, cutting device interface 370, implant database 380, and cutting block database 390. The input devices 360 can be configured and implemented to receive a surgical plan from the CAS system 320 for attaching an implant to a bone, for example. The surgical plan can utilize a cutting block to guide a cutting tool to remove a portion of the bone to facilitate accurate placement of the implant. The surgical plan can be utilized by the processor 340, immediately, or can be stored in memory 350 for later use. It should be understood that the tracking device 305 can configure and implement a single database that can be partitioned into the implant database 380 and the cutting block database 390.

The tracking device 305 can configure and implement the processor 340 to access the implant database 380 to identify the implant (or implants) that are the subject of the surgical plan as generated by the CAS system 320. The processor 340 can obtain implant geometry for the implant(s) from the implant database 380. The processor 340 can determine the implant location relative to the bone from the surgical plan.

In certain implementations, the tracking device 305 can configure and implement the processor 340 to access the cutting block database 390. The processor 340 can utilize the cutting block database 390 to determine the cutting block position relative to the implant location for the surgical plan.

In some examples, based upon the relationship between the implant location and the cutting block position, the processor 340 can determine what portion of the patient's bone to remove for attachment of the cutting block in a proper position.

The tracking device 305 can configure and implement the cutting device interface 370 to track the cutting device 330 over an area of bone to be removed. The cutting device 330 can include any type of tracked tool, robotic hand-piece, or autonomous robot that facilitates the ability to resect bone according to the necessary position and orientation guided or governed by the tracking device 305.

The implant database 380 can include information for each implant and/or implant design family, including information for each size component. The information can include the 3D shape of the implant (e.g. STL data), mounting implant peg hole trajectories (defined in the coordinate space of the 3D shape data), metadata describing sizing and labelling information, and geometry information describing the position and orientation of planar cuts to be made with a saw in order to install the implant. Each implant in the implant database 380 can be correlated with one or more compatible cut guide or cutting block.

The cutting block database 390 can include information relating one or more cut guide or cutting block to compatible implants in the implant database 380. The cutting block database 390 can physically define saw cut trajectories relative to a particular cut guide or cutting block. The cut guide or cutting blocks can be pinned to the bone to hold them rigidly to the bone during the cut. The cutting block database 390 can include information relating to pin holes for a particular cut guide or cutting block.

The cutting block database 390 can contain trajectory and placement data relating to the pin holes for each cut guide or cutting block. The cutting block database 390 can contain coordinate transform information (either explicitly, through specification in the cutting block database 390, or implicitly through assumed common coordinate definitions). The coordinate transforms can describe an expected alignment for the cut guide or cutting block, as well as implant coordinate systems. The implant coordinate systems can indicate how cut trajectories can coincide with the necessary planar cuts for installation of the implant.

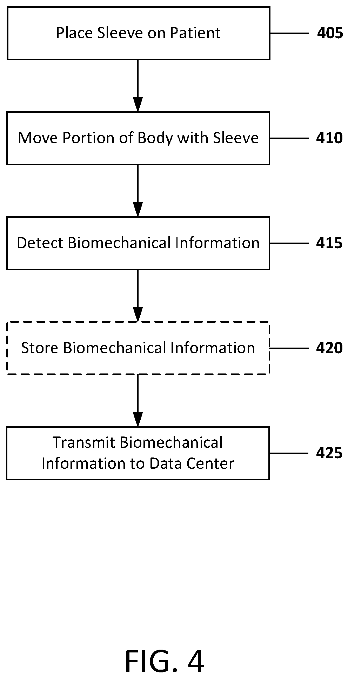

FIG. 4 depicts an illustrative flow diagram of an illustrative method of assessing and determining information pertaining to a patient in accordance with an embodiment of the present disclosure using, for example, a CAS system and navigation system as described above. The process of determining patient information may take place in an office of a healthcare provider, an ambulatory surgical center, a hospital, or a similar location at which healthcare services are provided.

As shown in FIG. 4, a sleeve such as sleeve 200 as described above may be placed 405 on a patient at a position of interest. For example, the sleeve may be placed 405 on, near or around a patient's knee, ankle, elbow, wrist, shoulder, hip, or the like. In an embodiment, the sleeve may be placed 405 in alignment with one or more features of the portion of the body on which it is placed. For example, the sleeve may include one or more markings that identify a proper position and/or orientation. In an alternate embodiment, the sleeve may be sized or shaped to achieve proper placement on the patient's body. For example, the sleeve can be appropriately sized and shaped to fit on a patient's knee.

In an embodiment, the patient may move 410 the portion of the body on which the sleeve is placed. For example, the patient may move 410 the sleeve by walking on a treadmill, riding a stationary bike, or the like for a sleeve placed on the patient's leg. Alternatively, the patient may move 410 his/her arm in a repeatable motion for a sleeve placed on the patient's arm.

A detection and/or navigation system may detect 415 biomechanical information related to the movements of the patient and/or the sleeve. For example, the detection and/or navigation system may detect 415 the movement of various components of the leg, such as the femur, the tibia, one or more ligaments, one or more muscles, or the like, while the patient is walking. In certain implementations, the detection and/or navigation system can detect 415 the biological information based upon information received from sensors in the sleeve itself, e.g., sensors 220 as described above. This information can include accelerometer data related to position and movement information collected by the sensors and, based upon this information, the detection and/or navigation system can detect 415 the biomechanical information.

In an embodiment, the biomechanical information may be stored 420 locally within a non-transitory computer-readable storage medium. In an embodiment, the biomechanical information may be transmitted 425 to a processing system for processing. For example, the biomechanical information may be transmitted to a data center, which determines various aspects of a surgical procedure based on the biomechanical information. In an embodiment, the biomechanical information may be used to identify one or more portions of a joint, such as a knee, that are more highly stressed during ambulation. This information may be used to select an implant device and/or identify one or more parameters such as optimal implant positioning for a surgical procedure. The implant device and/or identified parameters may be selected to, for example, alleviate stress within the knee. Alternate and/or additional information may be determined by the data center as will be apparent to one of ordinary skill in the art based on this disclosure.

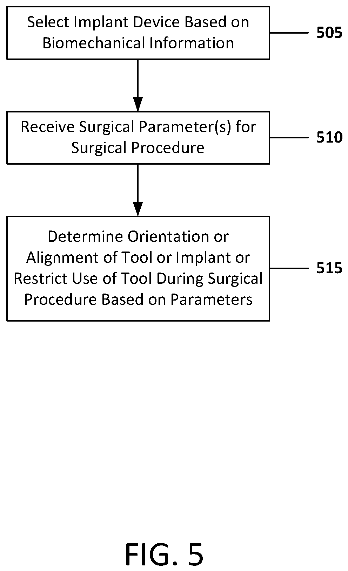

FIG. 5 depicts an illustrative flow diagram for selecting, placing and orienting an implant device in accordance with an embodiment. As shown in FIG. 5, an implant device may be selected 505 for a patient based on biomechanical information received from a pre-operative procedure, such as the process described above in reference to FIG. 4. The selected implant device may have one or more characteristics identified by a pre-operative processing system to best fit the patient, correct an abnormality in the patient, and/or the like.

One or more surgical parameters may be received 510 from a data center for use during the surgical procedure. The one or more surgical parameters may include, for example and without limitation, positioning information for the implant, an amount of bone to remove to accommodate the implant, an angle at which the implant should be oriented or for the cut used to remove bone, the location and orientation of a cutting guide block, and/or the like. In an embodiment, the one or more parameters may be received 510 by a detection and/or navigating system, including a camera system as disclosed above in reference to FIG. 3A, which monitors the position and orientation of a cutting tool including a plurality of tracking sensors during a surgical procedure.

In an embodiment, the one or more surgical parameters may be used to determine 515 whether certain aspects of a surgical procedure can be performed in order to provide the best outcome for the patient. In an embodiment, the detection and/or navigation system may use the one or more surgical parameters to determine 515 whether to restrict an ability of a cutting tool to operate. For example, the cutting tool may not be permitted to cut away a patient's bone unless and until the cutting tool is properly oriented and aligned. In another embodiment, the detection and/or navigation system may use the one or more surgical parameters to determine 515 whether to restrict operation of a boring tool. For example, the boring tool may be used to bore holes in a bone to enable pin placement for a cutting block. The boring tool may not be permitted to make such holes in a bone unless and until the boring tool is properly positioned and aligned. Other tools may be similarly restricted as will be apparent based on this disclosure.

In an embodiment, the determination of proper alignment and orientation of a tool may be determined 515 based on the alignment and orientation of the implant device to be implanted into the patient.

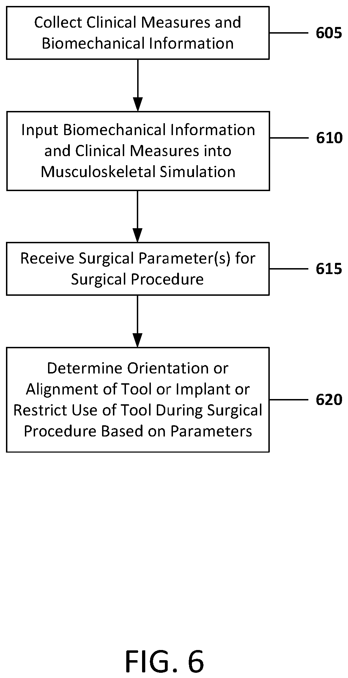

FIG. 6 depicts an alternative illustrative flow diagram for selecting, placing and orienting an implant device in accordance with an embodiment. As shown in FIG. 6, a system such as a CAS system can collect 605 various clinical measures and biomechanical information for a patient. For example, the biomechanical information can be used in conjunction with other clinical measurements including, but not limited to, anthropometric measurements, anatomical measurements derived from x-rays or magnetic resonance imaging, muscle activation patterns as determined by EMG or other tools, force-plate data, and other similar clinical measurements.

According to the invention, the system inputs the clinical measures and biomechanical information into a musculoskeletal simulation, namely a statistical shape model. In some examples, the statistical shape model can be generated using a plurality of images (e.g., from a bone atlas) of normal bones/tissue of comparable anatomical origin from a group of subjects having normal anatomy. The statistical shape model output is used in combination with shape morphing algorithms to create an accurate virtual representation of the patient's anatomy.

The clinically derived information, including 3D models, and/or biomechanical information can be input 610 as parameters in a musculoskeletal dynamic simulation. The simulation may be carried out using custom software created for this purpose or may be implemented with a commercial biomechanics simulation software such as LifeModeler. In such an example, physics based software can be used for biomechanical simulation and can provide estimation of the force environment inside the joint, including joint contact forces, shear forces, muscle strains, ligament tension, tendon strain, patellar tracking, and other similar forces. Recommendations for the surgical planning, physical therapy, medications, self-care, used of assistive devices, lifestyle modifications, and other disease treatments can be made based on the simulation results.

One or more surgical parameters may be received 615 for use during the surgical procedure based upon the simulations results. The one or more surgical parameters may include, for example and without limitation, positioning information for the implant, an amount of bone to remove to accommodate the implant, an angle at which the implant should be oriented or for the cut used to remove bone, the location and orientation of a cutting guide block, and/or the like. In an embodiment, the one or more parameters may be received 615 by a detection and/or navigating system, including a camera system as disclosed above in reference to FIG. 3A, which monitors the position and orientation of a cutting tool including a plurality of tracking sensors during a surgical procedure.

In an embodiment, the one or more surgical parameters may be used to determine 620 whether certain aspects of a surgical procedure can be performed in order to provide the best outcome for the patient. In an embodiment, the detection and/or navigation system may use the one or more surgical parameters to determine 620 whether to restrict an ability of a cutting tool to operate. For example, the cutting tool may not be permitted to cut away a patient's bone unless and until the cutting tool is properly oriented and aligned. In another embodiment, the detection and/or navigation system may use the one or more surgical parameters to determine 620 whether to restrict operation of a boring tool. For example, the boring tool may be used to bore holes in a bone to enable pin placement for a cutting block. The boring tool may not be permitted to make such holes in a bone unless and until the boring tool is properly positioned and aligned. Other tools may be similarly restricted as will be apparent based on this disclosure. In an embodiment, the determination of proper alignment and orientation of a tool may be determined 620 based on the alignment and orientation of the implant device to be implanted into the patient.

Information received from wearing a sleeve 205 may be used for additional purposes. For example, after a surgical procedure has been performed, a sleeve 205 may be placed on the patient at, near or around the location at which the implant device was implanted. The patient may perform the same or similar motions (i.e., ambulation or otherwise moving the extremity) as were performed pre-surgery. The one or more position trackers (210 in FIG. 2A) or the sensors (220 in FIG. 2B) may be used to determine the position and/or orientation of the sleeve 205 and/or of one or more of the patient's bones, ligaments, muscles, soft tissue, or the like in three-dimensional space as the patient's extremity moves. The post-operative tracking information may be compared with the pre-operative tracking information to determine one or more factors. For example, the comparison may enable a determination as to whether the surgery was successfully performed, a determination of a level of increased effectiveness of the joint as a result of the surgery, an assessment of whether rehabilitation has assisted in recovery, or other assessments of surgical quality. The post-operative tacking could be used to monitor the results of physical therapy.

The pre-operative and post-operative tracking information may be used as factors to determine ratings for one or more of a surgeon, implants, a hospital, a rehabilitation facility or the like with respect to the surgery and/or the post-operative care. For example, if the pre-operative tracking information disclosed a biomechanical issue in the patient and this issue was determined to be resolved through the surgery based on the post-surgical tracking information, the surgeon and the facility at which the surgery was performed may receive favorable ratings. Conversely, if the pre-operative tracking information resulted in the data center recommending removing 8 mm of bone from the tibia during a knee replacement surgery, the surgeon instead removed 10 mm of bone during the surgery, and the post-operative tracking information identified a gait issue in the patient, the surgeon and/or the facility at which the surgical procedure was performed may receive a negative rating. Such information may be provided to candidate patients for similar implant procedures to assist such patients in determining whether to have a procedure performed by a particular surgeon or at a particular hospital.

Alternately or additionally, the pre-operative and post-operative tracking information may be used to refine the algorithm used to assign the one or more surgical parameters via machine learning. For example, if the algorithm suggested a particular depth and angle for removing bone based on the pre-operative tracking information, the operation was performed successfully, and a gait issue is determined to be present post-surgery, the algorithm may be modified to provide different parameters for future surgical procedures. Alternately, a successful outcome may reinforce the assignment of one or more surgical parameters to a particular set of pre-operative conditions.

Payors, such as insurance companies or government agencies, could use the pre-operative and post-operative tracking information to objectively measure implants, surgeons, facilities, physical therapy, or other variables to guide purchasing decisions.

In the above detailed description, reference is made to the accompanying drawings, which form a part hereof. In the drawings, similar symbols typically identify similar components, unless context dictates otherwise.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (for example, bodies of the appended claims) are generally intended as "open" terms (for example, the term "including" should be interpreted as "including but not limited to," the term "having" should be interpreted as "having at least," the term "includes" should be interpreted as "includes but is not limited to," et cetera). While various compositions, methods, and devices are described in terms of "comprising" various components or steps (interpreted as meaning "including, but not limited to"), the compositions, methods, and devices can also "consist essentially of" or "consist of" the various components and steps, and such terminology should be interpreted as defining essentially closed-member groups. It will be further understood by those within the art that if a specific number of an introduced claim recitation is intended, such an intent will be explicitly recited in the claim, and in the absence of such recitation no such intent is present.

For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (for example, "a" and/or "an" should be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should be interpreted to mean at least the recited number (for example, the bare recitation of "two recitations," without other modifiers, means at least two recitations, or two or more recitations). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, "a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera. As a nonlimiting example, each range discussed herein can be readily broken down into a lower third, middle third and upper third, et cetera. As will also be understood by one skilled in the art all language such as "up to," "at least," and the like include the number recited and refer to ranges that can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

## Claims

1. A system for determining a position and orientation of an implant for a joint replacement surgery, the system comprising:
a sleeve (205) configured to be worn by a patient and to collect biomechanical information related to movement of the joint; and
a surgical system (320) configured to:
receive the collected biomechanical information, determine clinical measurement information related to the joint,
generate a musculoskeletal simulation for the joint based upon the collected biomechanical information and the determined clinical measurement information,
determine one or more surgical parameters for the patient based upon the musculoskeletal simulation,
determine at least one of a position and orientation of an implant component to be inserted into the joint based upon the one or more surgical parameters,
wherein the surgical system is further configured to generate a statistical shape model based upon the clinical measurement information and the biomechanical information,
wherein the surgical system is further configured to apply a shape morphing algorithm to the statistical shape model to generate an accurate virtual anatomical representation for the patient, and
wherein the biomechanical data comprises at least one of ligament movement information and muscle movement information for the joint as collected by the sleeve (205).

2. The system of claim l, wherein the sleeve (205) comprises one or more sensors (220) configured to determine a position of the sleeve.

3. The system of claim 1 or 2, wherein the sleeve (205) further comprises one or more position trackers (210) configured to be optically detected by a navigation system (300).

4. The system of any of preceding claims 1-3, wherein the one or more surgical parameters comprises at least one of position information for the implant component, an amount of bone to remove to accommodate the implant component, an angle at which the implant component is to be oriented, and a location and orientation of a cutting guide.

5. The system of any of preceding claims 1-4, wherein the clinical measurement information comprises at least one of anthropometric measurements, anatomical measurements, muscle activation pattern information, and force-plate data; and/or optionally the biomechanical data comprises at least one of bone position information, and other soft tissue information for the joint as collected by the sleeve (205).

6. A method for determining a position and orientation of an implant for a joint replacement surgery, the method comprising:
collecting, by a sleeve (205) configured to be worn by a patient, biomechanical information related to movement of the joint;
receiving, by a surgical system (320), the collected biomechanical information from the sleeve; determining, by the surgical system, clinical measurement information related to the joint;
generating, by the surgical system (320), a musculoskeletal simulation for the joint based upon the collected biomechanical information and the determined clinical measurement information;
determining, by the surgical system (320), one or more surgical parameters for the patient based upon the musculoskeletal simulation; and
determining, by the surgical system (320), at least one of a position and orientation of an implant component to be inserted into the joint based upon the one or more surgical parameters,
generating a statistical shape model based upon the clinical measurement information and the biomechanical information; and
applying a shape morphing algorithm to the statistical shape model to generate an accurate virtual anatomical representation for the patient,
wherein the biomechanical data comprises at least one of ligament movement information and muscle movement information for the joint as collected by the sleeve (205).

7. The method of claim 6, further comprising generating, by the surgical system (320), a statistical shape model based upon the clinical measurement information and the biomechanical information; and optionally applying, by the surgical system, a shape morphing algorithm to the statistical shape model to generate an accurate virtual anatomical representation for the patient.

8. The method of claim 6 or 7, wherein the one or more surgical parameters comprises at least one of position information for the implant component, an amount of bone to remove to accommodate the implant component, an angle at which the implant component is to be oriented, and a location and orientation of a cutting guide.

9. The method of any of preceding claims 6-8, wherein the clinical measurement information comprises at least one of anthropometric measurements, anatomical measurements, muscle activation pattern information, and force-plate data; and/or optionally the biomechanical data comprises at least one of bone position information and other soft tissue information for the joint as collected by the sleeve.

## Patentansprüche

1. System zum Bestimmen einer Position und Ausrichtung eines Implantats für Gelenkersatzoperationen, wobei das System umfasst:
eine Manschette (205), die dazu ausgelegt ist, von einem Patienten getragen zu werden und biomechanische Informationen zu Bewegungen des Gelenks zu sammeln; und
eine chirurgisches System (320), das hierzu ausgelegt ist:
Empfangen der gesammelten biomechanischen Informationen, Bestimmen klinischer Messinformationen zu dem Gelenk,
Generieren einer muskuloskelettalen Simulation für das Gelenk basierend auf den gesammelten biomechanischen Informationen und den bestimmten klinischen Messinformationen,
Bestimmen eines oder mehrerer chirurgischer Parameter für den Patienten basierend auf der muskuloskelettalen Simulation,
Bestimmen von wenigstens einem von einer Position und einer Ausrichtung einer Implantatkomponente, die in das Gelenk eingesetzt werden soll, basierend auf den ein oder mehreren chirurgischen Parametern,
wobei das chirurgische System ferner dazu ausgelegt ist, ein statistisches Formmodell basierend auf den klinischen Messinformationen und den biomechanischen Informationen zu generieren,
wobei das chirurgische System ferner dazu ausgelegt ist, einen Formwandlungsalgorithmus auf das statistisches Formmodell anzuwenden, um eine genaue virtuelle anatomische Darstellung für den Patienten zu generieren, und
wobei die biomechanischen Daten wenigstens eines von Ligamentbewegungsinformationen und Muskelbewegungsinformationen für das Gelenk umfassen, die von der Manschette (205) gesammelt werden.

2. System nach Anspruch 1, wobei die Manschette (205) einen oder mehrere Sensoren (220) umfasst, die dazu ausgelegt sind, eine Position der Manschette zu bestimmen.

3. System nach Anspruch 1 oder 2, wobei die Manschette (205) ferner einen oder mehrere Positionstracker (210) umfasst, die dazu ausgelegt sind, optisch von einem Navigationssystem (300) detektiert zu werden.

4. System nach einem der vorstehenden Ansprüche 1-3, wobei die ein oder mehreren chirurgischen Parameter wenigstens eines von Positionsinformationen für die Implantatkomponente, eine zu entfernende Knochenmenge, um die Implantatkomponente unterzubringen, einen Winkel, in dem die Implantatkomponente ausgerichtet werden soll, und einen Ort und eine Ausrichtung einer Schnittführung umfassen.

5. System nach einem der vorstehenden Ansprüche 1-4, wobei die klinischen Messinformationen wenigstens eines von anthropometrischen Messungen, anatomischen Messungen, Muskelaktivierungsmuster-Informationen und Kraftplattendaten umfassen; und/oder optional die biomechanischen Daten wenigstens eines von Knochenpositionsinformationen und anderen Weichgewebeinformationen für das Gelenk umfassen, die von der Manschette (205) gesammelt werden.

6. Verfahren zum Bestimmen einer Position und Ausrichtung eines Implantats für Gelenkersatzoperationen, wobei das Verfahren umfasst:
Sammeln, durch eine Manschette (205), die dazu ausgelegt ist, von einem Patienten getragen zu werden, von biomechanischen Informationen zu Bewegungen des Gelenks;
Empfangen, durch ein chirurgisches System (320), der gesammelten biomechanischen Informationen von der Manschette; Bestimmen, durch das chirurgische System, von klinischen Messinformationen zu dem Gelenk;
Generieren, durch das chirurgische System (320), einer muskuloskelettalen Simulation für das Gelenk basierend auf den gesammelten biomechanischen Informationen und den bestimmten klinischen Messinformationen;
Bestimmen, durch das chirurgische System (320), eines oder mehrerer chirurgischer Parameter für den Patienten basierend auf der muskuloskelettalen Simulation; und
Bestimmen, durch das chirurgische System (320), von wenigstens einem von einer Position und einer Ausrichtung einer Implantatkomponente, die in das Gelenk eingesetzt werden soll, basierend auf den ein oder mehreren chirurgischen Parametern,
Generieren eines statistischen Formmodells basierend auf den klinischen Messinformationen und den biomechanischen Informationen; und
Anwenden eines Formwandlungsalgorithmus auf das statistische Formmodell, um eine genaue virtuelle anatomische Darstellung für den Patienten zu generieren,
wobei die biomechanischen Daten wenigstens eines von Ligamentbewegungsinformationen und Muskelbewegungsinformationen für das Gelenk umfassen, die von der Manschette (205) gesammelt werden.

7. Verfahren nach Anspruch 6, ferner umfassend das Generieren, durch das chirurgische System (320), eines statistischen Formmodells basierend auf den klinischen Messinformationen und den biomechanischen Informationen; und optionales Anwenden, durch das chirurgische System, eines Formwandlungsalgorithmus auf das statistische Formmodell, um eine genaue virtuelle anatomische Darstellung für den Patienten zu generieren.

8. Verfahren nach Anspruch 6 oder 7, wobei die ein oder mehreren chirurgischen Parameter wenigstens eines von Positionsinformationen für die Implantatkomponente, eine zu entfernende Knochenmenge, um die Implantatkomponente unterzubringen, einen Winkel, in dem die Implantatkomponente ausgerichtet werden soll, und einen Ort und eine Ausrichtung einer Schnittführung umfassen.

9. Verfahren nach einem der vorstehenden Ansprüche 6-8, wobei die klinischen Messinformationen wenigstens eines von anthropometrischen Messungen, anatomischen Messungen, Muskelaktivierungsmuster-Informationen und Kraftplattendaten umfassen; und/oder optional die biomechanischen Daten wenigstens eines von Knochenpositionsinformationen und anderen Weichgewebeinformationen für das Gelenk umfassen, die von der Manschette gesammelt werden.

## Revendications

1. Système permettant de déterminer une position et une orientation d'un implant pour une chirurgie de remplacement d'articulation, le système comprenant :
un manchon (205) configuré pour être porté par un patient et pour recueillir des informations biomécaniques relatives au mouvement de l'articulation ; et
un système chirurgical (320) configuré pour :
recevoir les informations biomécaniques recueillies, déterminer des informations de mesure clinique liées à l'articulation,
générer une simulation musculo-squelettique de l'articulation sur la base des informations biomécaniques recueillies et des informations de mesure clinique déterminées,
déterminer un ou plusieurs paramètres chirurgicaux pour le patient sur la base de la simulation musculo-squelettique,
déterminer au moins une d'une position ou d'une orientation d'un composant d'implant à insérer dans l'articulation sur la base du ou des paramètres chirurgicaux,
le système chirurgical étant en outre configuré pour générer un modèle de forme statistique sur la base des informations de mesure clinique et des informations biomécaniques,
le système chirurgical étant en outre configuré pour appliquer un algorithme de morphage de forme au modèle de forme statistique afin de générer une représentation anatomique virtuelle précise pour le patient, et
les données biomécaniques comprenant au moins des informations parmi des informations sur le mouvement ligamentaire et/ou des informations sur le mouvement musculaire pour l'articulation telles qu'elles sont recueillies par le manchon (205).

2. Système selon la revendication 1, le manchon (205) comprenant un ou plusieurs capteurs (220) configurés pour déterminer une position du manchon.

3. Système selon la revendication 1 ou 2, le manchon (205) comprenant en outre un ou plusieurs dispositifs de poursuite de position (210) configurés pour être détectés optiquement par un système de navigation (300).

4. Système selon l'une quelconque des revendications précédentes 1 à 3, le ou les paramètres chirurgicaux comprenant au moins un élément parmi des informations de position pour le composant d'implant, une quantité d'os à enlever pour loger le composant d'implant, un angle selon lequel le composant d'implant doit être orienté, et un emplacement et une orientation d'un guide de coupe.

5. Système selon l'une quelconque des revendications précédentes 1 à 4, les informations de mesure clinique comprenant au moins un élément parmi des mesures anthropométriques, des mesures anatomiques, des informations de motif d'activation musculaire et des données de plaque de force ; et/ou éventuellement les données biomécaniques comprenant au moins des informations parmi des informations de position osseuse et d'autres informations de tissu mou pour l'articulation telles qu'elles sont recueillies par le manchon (205).

6. Procédé de détermination d'une position et d'une orientation d'un implant pour une chirurgie de remplacement d'articulation, le procédé comprenant :
le recueil, au moyen d'un manchon (205) configuré pour être porté par un patient, d'informations biomécaniques relatives au mouvement de l'articulation ;
la réception, par un système chirurgical (320), des informations biomécaniques recueillies à partir du manchon ; la détermination, par le système chirurgical, des informations de mesure clinique relatives à l'articulation ;
la génération, par le système chirurgical (320), d'une simulation musculo-squelettique pour l'articulation sur la base des informations biomécaniques recueillies et des informations de mesure clinique déterminées ;
la détermination, par le système chirurgical (320), d'un ou plusieurs paramètres chirurgicaux pour le patient sur la base de la simulation musculo-squelettique ; et
la détermination, par le système chirurgical (320), d'au moins une position et/ou une orientation d'un composant d'implant à insérer dans l'articulation sur la base du ou des paramètres chirurgicaux,
la génération d'un modèle de forme statistique sur la base des informations de mesure clinique et des informations biomécaniques ; et
l'application d'un algorithme de morphage de forme au modèle de forme statistique pour générer une représentation anatomique virtuelle précise pour le patient,
les données biomécaniques comprenant au moins des informations parmi des informations sur le mouvement ligamentaire et/ou des informations sur le mouvement musculaire pour l'articulation telles qu'elles sont recueillies par le manchon (205).

7. Procédé selon la revendication 6, comprenant en outre la génération, par le système chirurgical (320), d'un modèle de forme statistique sur la base des informations de mesure clinique et des informations biomécaniques ; et l'application facultative, par le système chirurgical, d'un algorithme de morphage de forme au modèle de forme statistique pour générer une représentation anatomique virtuelle précise pour le patient.

8. Procédé selon la revendication 6 ou 7, le ou les paramètres chirurgicaux comprenant au moins un élément parmi des informations de position pour le composant d'implant, une quantité d'os à retirer pour loger le composant d'implant, un angle selon lequel le composant d'implant doit être orienté, et un emplacement et une orientation d'un guide de coupe.

9. Procédé selon l'une quelconque des revendications précédentes 6 à 8, les informations de mesure clinique comprenant au moins un élément parmi des mesures anthropométriques, des mesures anatomiques, des informations de motif d'activation musculaire et des données de plaque de force ; et/ou éventuellement les données biomécaniques comprenant au moins des informations parmi des informations de position osseuse et d'autres informations de tissu mou pour l'articulation telles qu'elles sont recueillies par le manchon.
